# EUROPEAN PATENT APPLICATION

(11) **EP 4 483 791 A1**
(43) Date of publication of application: **01.01.2025**
(21) Application number: 23181706.5
(22) Date of filing: 27.06.2023
(51) Int. Cl.: A61B 5/021, A61B 5/022, A61B 5/00

(54) **APPARATUS FOR USE IN NON-INVASIVE HEMODYNAMIC PARAMETER MEASUREMENT**

(71) Applicant: Koninklijke Philips N.V., 5656 AG Eindhoven (NL)
(72) Inventor: SCHIPPER, Niels, Eindhoven (NL); YAU, Kam Hing, Eindhoven (NL); LOUWSMA, Hendrik Klaas, 5656AG Eindhoven (NL); VAN DE VEEN, Egbert, 5656AG Eindhoven (NL); WESSELS, Arnoldus Cornelis, 5656AG Eindhoven (NL); VAN DER VLIS, Peter, 5656AG Eindhoven (NL); DUINEVELD, Paulus Cornelis, 5656AG Eindhoven (NL); AMINE EL SAYED, Achraf, 5656AG Eindhoven (NL); TAMMINGA, Stephanus Jacob Gerardus, 5656AG Eindhoven (NL)
(74) Representative: Philips Intellectual Property & Standards

(57) **Abstract**

Provided is a cuff for use in hemodynamic parameter measurement, the cuff comprising a shell portion for surrounding a body part of a subject and a pressure application means surrounding the shell portion, such that the shell portion is disposed between the actuator and the body part. The shell portion is formed so as to comprise at least two circumferential sections which, when the cuff is assembled on the subject's body part, together form a closed loop around the subject's body part. The shell portions for example overlap at their respective ends, to form at least a first overlap region and a second overlap region. By forming the shell structure with multiple circumferential sections, this permits opening of the shell from the side by sliding apart the shell pieces at least at one tangential meeting point between the pieces. For example, in some embodiments, the shell structure may be opened by tangentially sliding the at least two shell portions apart at a first overlap area.

## Description

### FIELD OF THE INVENTION

The present invention relates to the field of non-invasive hemodynamic parameter measurement.

### BACKGROUND OF THE INVENTION

The most common method for non-invasive blood pressure (NIBP) measurement is the use of an oscillometric blood pressure measurement cuff. The cuff comprises an inflatable bladder. The cuff wraps around the upper arm of a patient and is controlled to progress through an inflation and deflation cycle during which a pressure inside the bladder is sampled and a single set of values for systolic arterial pressure and diastolic arterial pressure are derived through processing of the pressure signal.

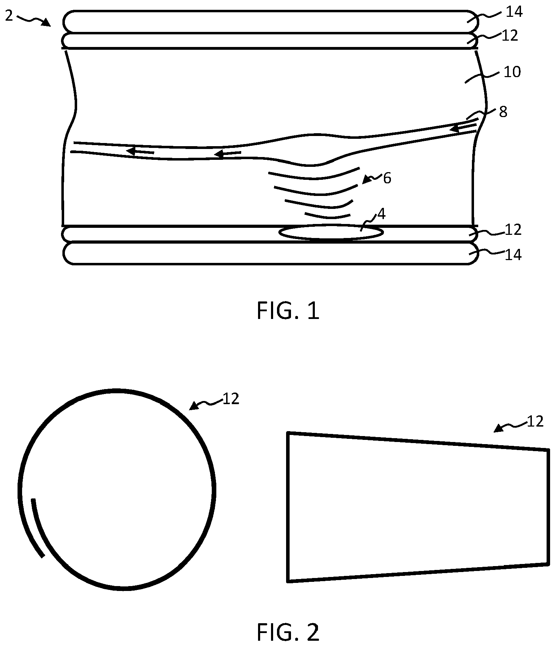

A variant approach to non-invasive blood pressure measurement has previously been proposed. This also uses a cuff which wraps around the body part of the patient. A cross-sectional illustration is shown in Fig. 1. The cuff 2 is shown wrapped around the arm 10 of a patient, and the brachial artery 8. The cuff 2 includes a pressure actuator 14, which optionally may be an inflatable bladder, as in the traditional NIBP cuff. In variance with the traditional NIBP cuff, the variant cuff also includes a separate tissue pressure sensor 4 arranged to measure a pressure between the body part 10 and the cuff. The tissue pressure sensor in some examples comprises a fluid-filled sensor pad, wherein a pressure in the pad varies as a function of pressure exerted on the pad. Also, in variance with the traditional NIBP cuff, the variant cuff includes a hard shell 12 which extends around the body part and is disposed (radially) between the actuator 14 and the body part 10.

The pulse waves in the brachial artery 8 cause a change in the skin movement. This change in skin movement causes a compression of the liquid in the sensor pad 4. The sensor pad is fluidly connected via a flexible tube filled with similar liquid to a pressure transducer which measures the pressure signal.

The pressure sensor pad 8 is enclosed by a hard shell 12 to enable stiffness and enable a sufficient signal from the pressure sensor. In similarity with a standard NIBP cuff, the pressure actuator 14 which may comprise a bladder which is inflatable with an air pump, to compress the arm tissue, so that the brachial artery 8 can be closed.

Due to the inclusion of the separate tissue pressure sensor 4 with hard shell 12 backing, this cuff can obtain a real time pulse signal for the patient, and, from this, multiple different hemodynamic parameters can be obtained, including stroke volume and cardiac output.

In the current state of the art, the shell structure 12 has a tangentially continuous structure, and, in the most common design, is formed from a single piece of material which is curled round to define a complete (closed) loop. Fig. 2 schematically shows an example shell structure 12 according to the state of the art. Fig. 2 (left) shows a cross-section across a plane normal to a longitudinal axis of the shell and parallel with tangential and radial axes of the shell. Fig. 2 (right) shows a cross-section across a plane parallel with a longitudinal axis of the shell. It can be seen that the shell structure is tangentially continuous.

In the current state of the art, there are multiple different cuff sizes to accommodate different arm diameters of patients, due to the fact that the rigid shell 12 is relatively inflexible in terms of the arm diameter that it fits to.

EP2953528B1 describes one example implementation of the above-described variety of blood pressure measurement cuff.

Suitable algorithms for processing the tissue pressure measurements to derive one or more hemodynamic parameters can be found for example in the documents: WO 2018/210931, WO 2020/148137, WO 2019/211210, US10485432, EP2759258B1, and US10349849.

For example, and as described in WO 2018210931, in some embodiments, values for SAP, DBP and MAP can be obtained based on identifying a peak in the amplitude of a series of measured tissue pressure pulses. The various blood pressure measures can then be determined based on identifying amplitude values of the tissue pressure pulse sequence at pre-defined reduced proportions of the peak amplitude value. The reader is referred to WO 2018/210931 for more details.

### SUMMARY OF THE INVENTION

It is the recognition of the inventors that a problem with the current shell-backed measurement cuff described above is the relative impracticality of mounting the cuff to the arm of the patient. Due to the presence of the rigid shell structure, which has a one-piece coil structure, the cuff has to be slid onto the arm via the hand. However, in practice, the hand may be cannularised for intravenous infusion. Thus, to fit the cuff requires first ceasing the infusion and removing the cannula. In addition, the need to slide the cuff onto the arm via the hand is physically challenging for the caregiver. Due to the tangentially closed architecture of the shell, to fit the cuff, the arm first has to be raised by the caregiver, and then, while holding the arm, the cuff itself must be slid into position. However, positioning the cuff correctly requires two hands. This means that, in practice, to apply the shell-backed cuff on a patient's arm, at least two caretakers are required.

The inventors have devised a development of the known shell-backed cuff which is aimed at addressing one or more of the above-identified problems.

The invention is defined by the claims.

According to examples in accordance with an aspect of the invention, there is provided an apparatus for use in non-invasive hemodynamic parameter measurement, comprising: a cuff for extending around a body part of a subject, wherein the cuff comprises a pressure actuator for applying a controllable pressure to the body part, and further comprises a shell structure for extending around the body part and, when the cuff is worn, is disposed between the actuator and the body part.

The shell structure is configurable in each of a closed state and an open state.

In the closed state, the shell structure forms a closed loop around a central lumen, the central lumen for receiving the body part of a subject, and wherein, in the open state, an opening/break/discontinuity is in a circumference of the shell structure to permit insertion of the arm into the lumen.

The shell structure is composed of at least two shell portions (at least a first shell portion and at least a second shell portion), each for defining a respective portion of the circumference of the shell when the shell is in the closed state, the shell portions overlapping tangentially in the closed state across at least a first overlap area and a second overlap area.

Transition of the shell structure from the closed state to the open state comprises tangentially separating the shell portions at least at one of the overlap areas.

Thus, it is proposed to provide a hemodynamic measurement cuff with a shell structure, and wherein the shell structure has two circumferential breaks (discontinuities), allowing it to be fitted onto an arm sideways or laterally instead of needing to thread the arm through the central lumen from the hand up to the upper arm. This makes the cuff easier to fit. In particular, fitting can be performed by a single caregiver: one hand can be used to lift and hold the arm of the patient, while the other hand can be used to attach the cuff and position it on the arm.

Furthermore, a supplementary benefit of the proposed design is simplified manufacturing. The state-of-the-art shell-backed cuff comprises a shell structure formed of a single piece which is coiled around in manufacture to form a tubular shape. This requires a complicated molding process. According to embodiments of the present invention, since there are two or more shells portions, these can be formed more simply, for example using a simple open-close injection-molding process.

In some embodiments, a releasable securement means may be provided at the first overlap area for, when secured, preventing lateral sliding between the first and second shell portions in a tangential direction at the first overlap area when the shell is in the closed state. Providing means for tangentially securing the two shell portions at one of the two overlap areas enables the multi-piece design to functionally replicate or mimic the architecture of the tangentially continuous shell of the prior art. In particular, for optimal measurement accuracy, it is best to have only a single area of tangential sliding overlap.

This is for several reasons. A first is that, preferably, when the cuff is mounted to the arm in use, the brachial artery is located adjacent to a point of the shell which is immovable radially, such that changes in pressure in the artery are maximally coupled to the sensor pad. Thus it is preferable that the sensor pad is disposed beneath a section of the shell which does not slide tangentially, so that artery pressure changes can be sensed and are not simply absorbed by sliding of the shell portions. Thus, providing only one area of sliding overlap minimizes artery pressure signal loss as measured at the sensor pad.

Further, it is beneficial to have only one sliding overlap area as each sliding overlap area carries the risk of skin pinching and/or extra friction between the moving pieces of the shell.

However, securement of one of the two overlap areas is not essential. For example, the inventors have found that a similar effect can be achieved by providing the two shell portions with different stiffnesses. The stiffnesses can be tuned so that, in operation, when radial force is applied to the shell structure from the body part, the shell consistently preferentially accommodates the force by movement at only one of the two overlap areas.

A further advantage of providing the releasable securement means at the first overlap area is that, optionally, the securement means may be provided so as to permit securement of the first and second shell portions at the first overlap area at a plurality of different relative tangential positions. This advantageously allows for adjusting a diameter of the shell (when in the closed state), and therefore also the overall cuff, to accommodate different arms sizes.

By way of just one example, the securement means may comprise a hook-and-loop fastening (i.e., Velcro°).

In some embodiments, at least at the second overlap area, the first and second shell portions are freely laterally slidable in a tangential direction when the shell is in the closed state. Preferably, the first and second shell portions are freely laterally slidable only at the second overlap area and not at the first overlap area. Thus, an overlap area which accommodates tangential sliding is provided. Preferably, this is combined with the releasably securable first overlap area, so that, in operation of the device, there is provided a shell structure with a fixed overlap area (no tangential sliding) and a moveable overlap area (permits tangential sliding). This therefore approximately replicates the architecture of the state of the art shell but with the benefit of the various improvements in terms of practicality and ease of manufacture outlined above.

In some embodiments, the pressure actuator is arranged, when the cuff is in use on the body part, so as to radially support the shell structure against radial separation of the at least two shell portions. In other words, the pressure actuator acts to help hold the pieces of the shell structure together. The pressure actuator may for example be arranged to extend tangentially around the outside of the shell structure. It may be attached to an outside of the shell structure in one or more places.

In some embodiments, the apparatus further comprises a sheet article arranged to be disposed, when the cuff is applied to the arm, between the shell structure and a surface of the body part, and arranged for contacting the body part during use, and wherein the shell structure is slidable tangentially relative to the sheet article. In other words, a liner is provided between inner surfaces of the shell structure and the body part. This helps prevent pinching or wrinkling of the skin when the shell moves tangentially. The shell can slide over the liner.

In some embodiments, the sheet article may be provided attached to the shell structure. The alternative configuration is to provide the sheet article as a separate item which is independently wrapped around the body part by a caregiver before fitting the cuff. By attaching the sheet article, the separate fitting step is avoided. To facilitate this, in some embodiments, the sheet article is arranged to extend continuously around a radially interior face of the shell structure between a first attachment point on the first shell portion and a second attachment point on the second shell portion, and preferably wherein the first and second attachment points are located at the first overlap area.

The sheet article may extend uninterrupted over/across the second overlap area and may be discontinuous at the first overlap area, wherein a first end is attached to the first shell portion at the first overlap area and the second end is attached to the second shell portion at the first overlap area. In this way, the sheet article extends around an interior face of the shell structure and is held taught by the two separable ends of the first and second shell portions.

This is an efficient way of attaching the sheet article. This has the benefit of still enabling, when the cuff is worn, relative tangential sliding between the sheet article and the shell, since the liner is only attached to the shell at its two ends. It also has the further advantage that, with this arrangement, the sheet article effectively performs a secondary function of at least partially holding the two shell portions together at the second overlap area. In particular, the two shell portions cannot fully separate from one another, since the sheet article, attached to both the first and second shell portions, links the two together. As will become clear from the descriptions later, this also makes fitting the cuff to the patient much easier, since the two pieces of the shell can be effectively hinged open while remaining as a single structure which can be handled with one hand.

In some embodiments, one of the at least two shell portions has a greater stiffness than the other of the two shell portions. This has the advantage of effectively enforcing a preferential direction of tangential motion between the two shell portions at any of the overlap areas. In particular, the more flexible shell portion will tend to be more sensitive to radial pressure exerted thereon than the less flexible portion, so that the more flexible portion will tend to move relative to the arm to accommodate these pressure changes, while the less flexible portion stays relatively static relative to the arm. This has the advantage of maintaining consistency in the motion behavior of the shell during operation.

In some embodiments, one of the two shell portions may be formed of a different material to the other of the two shell portions, for example a material of a different stiffness or elasticity to the other of the two shell portions.

For example, in some embodiments, the apparatus further comprises a tissue pressure sensor for sensing a pressure between the cuff and the body part when the cuff is worn, the tissue pressure sensor comprising a sensing pad for disposal, in use, between the shell structure and the body part. This pressure sensor pad can be located between the stiffer shell portion and the body part, meaning that, in operation, sliding movement of the shell does not impact pressure readings from the sensor pad because all of these motions are accommodated by the less stiff shell portion. Further, it is advantageous to have a stiffer shell in close vicinity to the brachial artery, and hence the sensor pad, to ensure that the loss of signal from the brachial artery is minimal.

In some embodiments, the shell structure includes a first shell portion and a second shell portion, and wherein, at both the first and second overlap regions, the second shell portion is disposed radially above/atop the first shell portion. This therefore forms a configuration with a lower and upper shell. In some embodiments, the second shell portion (the upper shell portion) is less stiff than the first shell portion (the lower shell portion). Thus, in this set of embodiments, the first shell portion is the radially lower shell portion and the stiffer shell portion. For example, the different stiffness is helpful to facilitate an effect wherein the upper shell will, during sliding, follow the lower shell and not bend outwards.

In some embodiments, the first and second shell portions are formed of different materials, for example wherein the different materials have different stiffnesses.

As mentioned above, in some embodiments, the apparatus further comprises a tissue pressure sensor for sensing a pressure between the shell structure and the body part when the cuff is worn, the tissue pressure sensor comprising a sensing pad for disposal, in use, between the shell structure and the body part. In some embodiments, the tissue pressure sensor is arranged so as to be disposed between the first (lower) shell portion and the body part during use.

As mentioned above, the advantage of placing the sensor pad between the less flexible shell portion and the body part is that it can be expected to remain relatively static relative to the arm even as the shell structure varies in diameter via sliding at the second overlap area. This is because the more flexible shell structure will tend to kinetically absorb all of the sliding motion. Furthermore, the placement of the sensor pad radially adjacent the more stiff shell has the benefit of increased signal strength, since the shell has the effect of reducing signal loss from radial damping.

In some embodiments, the pressure actuator is attached, fixedly or removably, to the shell structure. The pressure actuator in some embodiments comprises an inflatable bladder. Other options are also possible such as a mechatronic actuator.

In some embodiments, the pressure actuator is for wrapping around the body part when the cuff is worn. The actuator may be attached or attachable at a first attachment zone to one of the two shell portions and optionally may be attached or attachable at a second attachment zone to a second of the two shell portions.

The actuator may be configurable in each of a closed state and an open state, wherein, in the closed state, the actuator forms a closed loop around the central lumen, and wherein, in the open state, an opening is defined in a circumference of the actuator to permit insertion of the arm into the lumen. Transition from the closed to open state may comprise unrolling the actuator to tangentially separate two ends of the actuator. Thus, here the actuator forms an additional annular layer extending circumferentially around the shell structure and which can be tangentially opened (e.g., unfolded or unrolled) to allow the cuff to be attached or removed from the arm.

The pressure actuator may comprise a length section which incorporates an inflatable bladder, and further may comprise at least one further length section without an inflatable bladder. The actuator may be for wrapping around a circumference of the shell structure, with the length extending around the circumferential dimension of the shell structure.

Although examples described above relate to an apparatus in which the shell structure comprises two shell portions, in further embodiments, the shell structure may comprise more than two shell portions, for instance three or more overlapping shell portions. Similar principles to those discussed above may be applied. For example, where three shell portions are provided, there may be two of the fixed overlap areas and one of the sliding overlap areas.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a cross-section through a prior art hemodynamic parameter measurement apparatus of which embodiments of the present invention represent a development;
Fig. 2 illustrates cross-sectional views of an example shell structure used in the apparatus of Fig. 1.
Fig. 3 shows the shell portion of an example apparatus in accordance with one or more embodiments in an open state for receipt of a body part of the subject;
Fig. 4 shows the shell portion of Fig. 3 in a closed state;
Fig. 5 illustrates in greater detail components of an example apparatus in accordance with one or more embodiments;
Fig. 6 illustrates an example apparatus in accordance with one or more embodiments, including a shell portion and pressure actuator;
Fig. 7 and Fig. 8 schematically illustrate an example structure and arrangement of a pressure actuator relative to the shell structure in accordance with one or more embodiments;
Fig. 9 illustrates different possible locations for the tissue pressure sensor pad within the apparatus; and
Fig. 10 illustrates a recessed channel in an interior face of the shell structure for accommodating a fluid tubing for a hydraulic tissue pressure sensor.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a cuff for use in hemodynamic parameter measurement, the cuff comprising a shell portion for surrounding a body part of a subject and a pressure application means surrounding the shell portion, such that the shell portion is disposed between the pressure application means and the body part. The pressure application means is operable to apply a variable pressure to the body part during a measurement cycle. The shell portion is formed so as to comprise at least two circumferential sections which, when the cuff is assembled on the subject's body part, together form an annular loop around the subject's body part. The shell portions for example overlap at their respective ends, to form at least a first overlap region and a second overlap region. By forming the shell structure with multiple circumferential sections, this permits opening of the shell from the side by pulling apart the shell pieces at least at one tangential meeting point between the pieces. For example, in some embodiments, the shell structure may be opened by tangentially pulling the at least two shell portions apart at a first overlap area.

Thus at least one aspect of the proposed apparatus is the provision of a cuff with a shell, and wherein the shell is not permanently tangentially closed, but is able to be tangentially opened around the arm. In at least one set of embodiments, this is achieved by providing the shell structure as a system of two tangentially overlapping shells, each defining a respective section of a tubular circumference. These can be opened during attachment of the cuff by pulling them apart tangentially, at least at one circumferential location. When the cuff is worn, the pieces can be pushed together to form a tangentially closed system around the arm circumference.

This enables the attachment of the cuff from the side of the arm. As discussed above, in state of the art versions of a shell-backed cuff, the shell cannot be opened tangentially, and thus the whole cuff must be mounted to the arm via the hand, and slid up to the correct location. This typically requires two caregivers to apply the cuff: one to hold the subject's arm, the other to slide on the cuff. Using the proposed apparatus, it is possible to apply the cuff with only a single caregiver.

By way of background, Fig. 1 shows a schematic illustration of the basic layered structure of a prior art hemodynamic parameter measurement apparatus of which embodiments of the present invention represent a development.

The apparatus can be used as part of a system or method for detecting blood pressure and/or other hemodynamic parameter measurements such as cardiac output or stroke volume. The illustration shows the cuff 2 applied to the upper arm 10 of a patient. An artery 8 of the patient is schematically shown. The apparatus has a design which differs from most standard cuff-based blood pressure measurement apparatuses in that it includes a dedicated tissue pressure sensor 4 which the cuff acts to hold against the tissue of the skin when the cuff is inflated. This feature is shared by embodiments of the present invention.

The cuff 2 includes a pneumatic pressure actuator 14 in the form of an inflatable bladder for use in changing a pressure applied to the body part 10 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled.

The cuff 2 further includes a shell structure 12, wherein the shell structure is arranged so as to be located between the pneumatic actuator 14 and the body part 10 when the cuff is worn on the body part, and arranged to surround the body part when the cuff is worn. The shell structure may be relatively stiff. Consequently, the measurement accuracy can be improved, since the pressure in the arm is measured by the tissue pressure sensor pad 4 against a relatively stiff support, which prevents dampening of the amplitude and shape of the signal. In particular, if the tissue pressure sensor pad 4 is located at least partially between the shell structure 12 and the body part 10, a high accuracy of the signals measured by the tissue pressure sensor unit can be achieved. With such a configuration of the tissue pressure sensor pad 4, the shell structure 12 does not absorb or attenuate the arterial pressure signal.

The apparatus of Fig. 1 differs from standard blood pressure measurement cuffs. Standard cuffs use the bladder air pressure as the sole measurement signal for sensing blood pressure. By additionally or alternatively utilizing a tissue pressure sensor 4, the apparatus shown in Fig. 1 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy > 90% of the amplitude and contour of a tissue pressure pulse wave. By contrast, by using a dedicated tissue pressure sensor, the design illustrated in Fig. 1 allows recording high-fidelity (HiFi) artery blood pressure and pulse waveform.

The tissue pressure sensor 4 may be implemented as, e.g., a fluid-filled bag which is fluidically coupled to a pressure transducer for read-out of a tissue pressure signal. When blood pulses in the artery 8, this results in a pressure wave 6 which can be detected by the tissue pressure sensor 4. The tissue pressure sensor 4 can be connected to the pressure transducer via a fluid-filled tube/line. The pressure transducer converts the pressure in the fluid into an electrical signal.

Thus, the operating principle is based on coupling of the pressure sensor to the body part (e.g. arm) in order to transcutaneously record tissue pressure pulse waves that result from arterial pulsation (e.g. brachial artery). In similar fashion to a conventional upper arm blood pressure cuff, the cuff compresses the upper arm using an integrated pneumatic actuator with rising clamping pressure. However, the actual compression may be achieved via the narrowing diameter of the rigid circumferential shell 12.

The shell structure 12 comprises a single unitary piece of stiff material which is curled in a loop shape with the two ends of the piece overlapping tangentially. This is illustrated schematically in Fig. 2.

Suitable algorithms for processing the tissue pressure measurements to derive hemodynamic parameters such as systolic arterial pressure (SAP), diastolic blood pressure (DAP), mean arterial pressure, and cardiac output can be found for example in the documents: WO 2018/210931, WO 2020/148137, WO 2019/211210, US10485432, EP2759258B1, and US10349849.

For example, and as described in WO 2018210931, in some embodiments, values for SAP, DBP and MAP can be obtained based on identifying a peak in the amplitude of a series of measured tissue pressure pulses. The various blood pressure measures can then be determined based on identifying amplitude values of the tissue pressure pulse sequence at pre-defined reduced proportions of the peak amplitude value. The reader is referred to WO 2018/210931 for more details.

Embodiments of the present invention differ from the known design, at minimum, in respect of the formation of the shell structure 12. As explained further below, it is proposed to provide a shell structure which is formed of at least two separate circumferential or arcuate pieces which overlap to define an overall arc or loop around the body part.

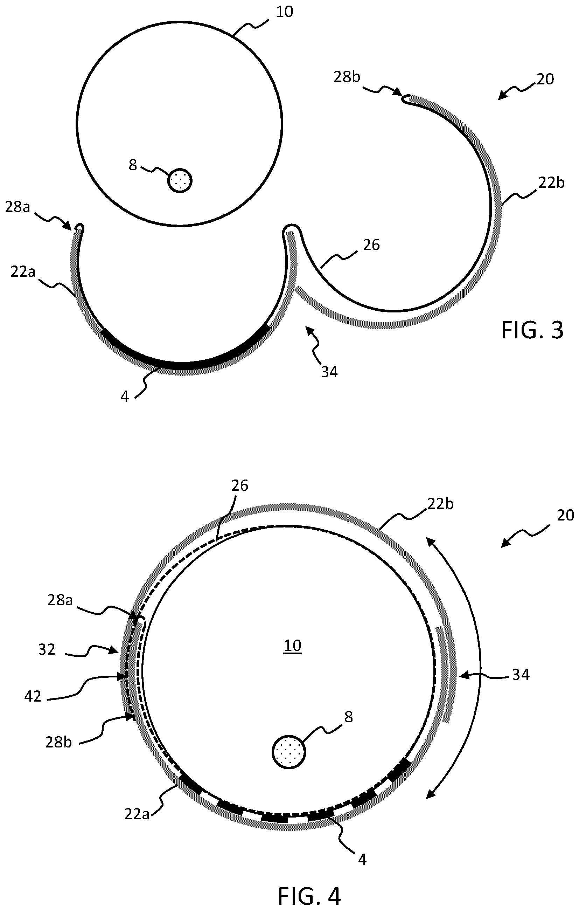

Figs. 3 and Fig. 4 schematically illustrate the principle according to one or more embodiments of the invention.

According to one or more embodiments, there is provided an apparatus for use in non-invasive hemodynamic parameter measurement.

The apparatus comprises a cuff 20 for extending around a body part 10 of a subject. For purposes of illustration, Fig. 3 shows the body part as the upper arm 10 of the subject. The brachial artery 8 is schematically indicated.

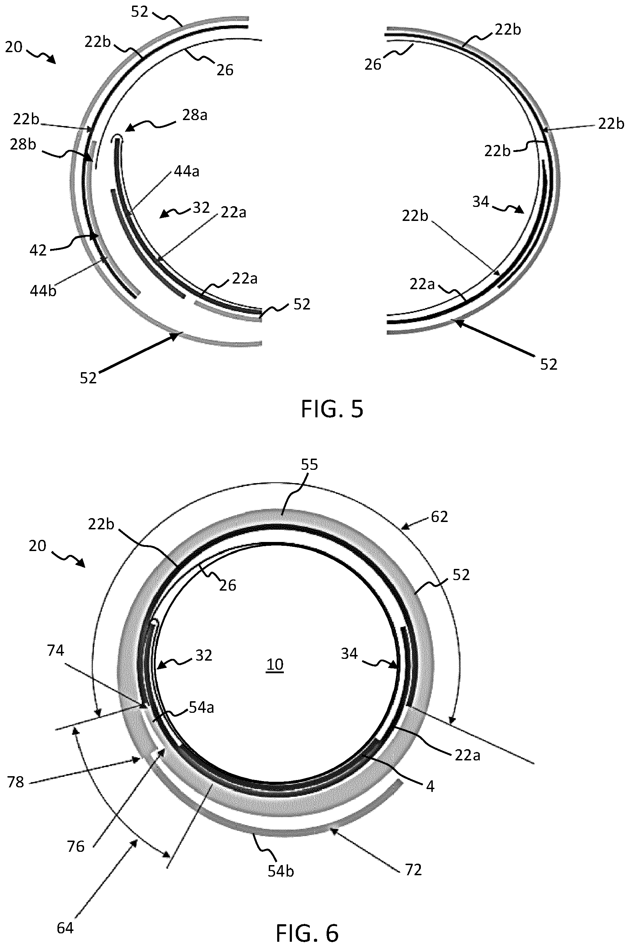

The cuff 20 comprises a pressure actuator 52 (see Fig. 5 and Fig. 6) for applying a controllable pressure to the body part 10. The cuff further comprises a shell structure 22 for extending around the body part 10 and is arranged such that, when the cuff is worn, the shell structure is disposed between the actuator and the body part. With reference to Fig. 3 and Fig. 4, the actuator would be applied radially atop the illustrated shell structure 22.

The shell structure 22 is configurable in a closed state and an open state. Fig. 3 illustrates the open state. Fig. 4 illustrates the closed state.

In the closed state, the shell structure 22 forms a closed loop around a central lumen, the central lumen for receiving the body part 10 of the subject.

In the open state, an opening/break/discontinuity is defined in a circumference or perimeter of the shell structure 22 to permit insertion of the arm into the lumen.

The shell structure 22 is composed of at least a first shell portion 22a and a second shell portion 22b, each for defining a respective portion of the circumference of the shell structure when the shell structure is in the closed state. The shell portions 22a, 22b overlap tangentially in the closed state to form at least a first overlap area 32 and a second overlap area 34.

The shell structure permits reversible transition between the open state and the closed state. As illustrated by Fig. 3, the transition from the closed state to the open state comprises separating the shell portions 22a, 22b at least at one of the overlap areas. In the illustrated example, the shell portions are separable at the first overlap area 32.

In the particular example illustrated in Fig. 3 and Fig. 4, the cuff further includes a liner formed of a sheet article 26 extending around a radially interior face of the shell structure. The liner has the effect of holding the two shell portions 22a, 22b together at one of the overlap areas 34, and whereby the transition from the closed to the open state can be achieved by hinging the two shell portions apart, wherein one of the overlap areas 34 acts as a pivot/hinge, and the two shell portions separate tangentially at the other 32 of the two overlap areas. The liner comprising the sheet article 26 is not essential.

As will be explained later, the primary function of the sheet article 26 is to provide a sliding interface between the skin and the shell, so that the shell can slide tangentially relative to the body part without wrinkling or pinching the skin. Instead, the shell slides over the liner 26 while the liner remains static relative to the skin.

In the illustrated example of Fig. 3, the apparatus further includes a tissue pressure sensor for sensing a pressure between the cuff and the body part when the cuff is worn, the tissue pressure sensor comprising a pressure sensing pad 4 for disposal, in use, between the shell structure 22 and the body part 10. For example, the pressure sensing pad 4 may be a fluid-filled pad and wherein the pressure sensor further includes a pressure transducer fluidly connected with the pad for sensing a pressure applied to the pad. Any other implementation of a pressure sensor is also possible.

Although it is not essential, in a preferred set of embodiments, and as illustrated in greater detail in Fig. 5, in some embodiments, there may further be provided a releasable securement means 42 at the first overlap area 32 for, when secured, preventing lateral sliding between the first 22a and second 22b shell portions in a tangential direction at the first overlap 32 area when the shell is in the closed state. Therefore, during operation, this overlap area is effectively fixed tangentially, whereby the two shell portions 22a, 22b are not able to slide over one another. On the other hand, at the second overlap area 34, the first 22a and second 22b shell portions are preferably freely laterally slidable in a tangential direction when the shell is in the closed state. Thus, this forms a sliding overlap area during operation, permitting the shell to expand or contract as the actuator applies less or more pressure. Application of pressure causes the volume of the arm to decrease, and the sliding overlap permits the cuff to accordingly decrease in diameter to match the changing diameter of the arm. If the shell portions do not slide to allow matching of the cuff to the arm diameter, a part of the brachial artery pressure signal would be lost to the sensor pad due to inefficient mechanical coupling of the artery to the sensor pad 4.

With regards to the fixable first overlap area 32, by providing this part fixed during measurement, signal strength is increased since this provides a firm backing support for the sensor pad, such that pressure changes in the artery are maximally coupled to the sensor pad. If the first overlap area were provided slidable, pressure changes in the artery could be at least partially absorbed by tangential sliding of the ends of the shell portions against one another, thereby reducing the strength of the measured arterial pressure signal.

Furthermore, in accordance with a preferred set of embodiments, transitioning from the closed state (Fig. 4) to the open state (Fig. 3) may comprise tangentially separating the two shell portions 22a, 22b at the first overlap area 32. This may therefore comprise first releasing the securement means 42 and then pulling/sliding apart the two shell portions 22a, 22b so that a break or opening in the shell circumference is defined at the location where the first overlap portion 32 formerly was.

In addition, preferably the releasable securement means 42 permits securement of the first 22a and second 22b shell portions at the first overlap area 32 at a plurality of different relative tangential positions. In other words, the circumferential length of the overlap between the two shell portions at the first overlap area may be adjusted. In this way, the diameter or circumference of the shell portion may be adjusted to the diameter or circumference of the subject's body part by simply attaching the two ends of the shell portions at the first overlap area, using the securement means, at a position where the shell is optimally fitted around the body part.

By way of one example, and as illustrated in Fig. 5, the securement means 42 may comprise a hook-and-loop fastening (i.e. Velcro^{®)}. Fig. 5 shows a means for implementing this at the first overlap area 32, by providing complementary hook-and-loop fastening surfaces 44a, 44b on the first shell portion 22a and second shell portion 22b respectively, these being arranged in facing relationship to permit coupling of the two shell portions together at the first overlap area 32. It is noted, that for ease of viewing and ease of labelling, the cuff is shown split in two halves. However, the cuff in reality is continuous as shown in Fig. 6.

If a hook-and-loop fastening is used, the shear strength of the loop surface may be between 8 and 30 PSI and more preferably between 15 and 23 PSI. The peel force strength of the loop is preferably between 0.2 and 4 pounds per inch width (PIW) and more preferably between 0.5 and 2 PIW. For the hook surface, the peel strength is preferably between 0.1 and 2 PIW and more preferably between 0.3 and 1 PIW.

Instead of a hook-and-loop fastening, other options may be used to fixate the first overlap area 32, such as a pushbuttons, magnets, or mechanical interlock via physically engaging members on the shell such a tie-wrap based construction.

Thus, it will be appreciated that the formation of the shell structure 20 with multiple shell portions enables a secondary benefit of allowing the working diameter of the overall shell structure to be adjustable. In particular, to arrive at a tangentially open construction, there is a requirement for a second shell portion which can be opened opposite to the sliding overlap area 34. As explained above, it is preferable that this openable overlap area be fixed in position during measurement for maximizing measured signal strength.

This gives the opportunity to provide functionality to adapt the tangential length of the overlap between the two shell portions at the fixed overlap area, so as to enable fitting to a wide variety of arm circumferences. A benefit of providing such an adaptive fixed overlap area is that the variation in arm circumference is captured by the fixed overlap instead of the sliding overlap, meaning that, for any of a range of different arm diameters, the overlap length of the slidable overlap area can be made approximately the same. Preferably, it allows the overlap length of the sliding overlap area to be made minimal, thereby reducing friction between the shells.

With regards to the second (slidable) overlap area 34, by way of illustrative example, the length of the overlap area in the tangential direction may be between 20-60 mm, and with an axial length (i.e. the dimension along the length of the lumen of the cuff) between the 80 and 180 mm and more preferably in the range of 120 and 160 mm. It is noted that these measurements are particularly suited for a cuff designed for use by adults. For smaller body sizes, different dimension may be needed.

With regards to the fixed overlap, this has preferable a minimum overlap length of 10 mm and more preferably in the range of 25 to 35 mm for a relatively big adult size arm. This might increase to a length in the range of 80-120 mm for a smallest expected adult arm.

As mentioned briefly above, in some embodiments, the apparatus comprises a sheet article 26 disposed, when the cuff is applied to the arm, between the shell structure 22 and a surface of the body part 10, and arranged for contacting the body part during use, and wherein the shell structure is slidable tangentially relative to the sheet article. In other words, a liner is provided between shell and body part. The shell can slide over the liner. This prevents pinching of the skin when the shell moves.

As illustrated in Fig. 3-5, the sheet article 26 is attached to the shell structure 22, and is arranged to extend continuously around a radially interior face of the shell structure between a first attachment point 28a on the first shell portion 22a and a second attachment point 28b on the second shell portion 22b. The first 28a and second 28b attachment points are located at the first overlap area 32.

In this example, the sheet article 26 extends uninterrupted over the second overlap area 34 and is discontinuous at the first overlap area 32. A first end of the sheet article is attached 28a to the first shell portion 22a at the first overlap area 32 and the second end is attached 28b to the second shell 22b portion at the first overlap area.

To explain further, compared to a prior art version of the shell-backed cuff, the multi-piece shell structure 22 offers the opportunity to simplify the liner. In particular, since the two shell portions 22a, 22b are positioned adjacent to one another, this allows provision of the liner in the form of the flat sheet 26 which is folded around the two shell portions. Compared with the liner design used in the current version of the shell-backed cuff (e.g. as is described in the document EP2953528B1), this simplifies manufacturing and improves usability. In particular, the liner used in known versions of the shell-backed cuff must be formed as closed loop, in order to be implemented with the tangentially closed shell structure. However, the tangentially open architecture of the proposed shell in accordance with embodiments of the present application allows for provision of the liner as a sheet article which is discontinuous, and having ends which are attached to the two shell portions.

During manufacturing, with the two shell portions positioned adjacent to one another, the sheet article 26 forming the liner may be laid atop the radial interior faces of the two shell portions, extended around the interior face of the shell portions and finally affixed at a first 28a and second 28b attachment point on the first 22a and second 22b shell portion respectively. The attachment may be made at a backside of each respective shell portion 22a, 22b. Thus the liner is effectively wrapped around the inside of the shell structure. The attachment may be with a removable fastening, such as Velcro° or a permanent fastening such as glue, or with mechanical hooks integrated in the shell portions.

With the provision of the sheet article 26 as described, the sheet article prevents pinching of skin due to movement of the shell structure 22, but also allows the shell structure to move freely around the arm. As mentioned above, the sheet article extends uninterrupted over the second (sliding) overlap area 34, meaning that the sliding between the two shell portions is not hampered by an interrupted liner.

The fully integrated liner described above is not essential. Instead, in some embodiments, a hybrid solution may be implemented, wherein the sheet article of the liner is applied to the body part in a separate step before the cuff 20 is applied. This may involve for example wrapping the sheet article around the body part and securing it in a circumferentially closed state. For example, the sheet article is fastened and unfastened to tangentially close and open it. For example, a suitable fastening may be a hook and loop fastening or self-adhesive material. The cuff 20 of the apparatus may in this case be the same as the previously discussed example, but excluding the sheet article 26.

For optimal sensing functionality, it is preferable to implement the two shell portions 22a, 22b with a configuration such that tangential sliding of the cuff over the arm during operation involves motion of only one of the two shell portions, while the other remains relatively static. In other words, one of the two shell portions moves preferentially over the other responsive to radial force. In this way, a master-slave relationship is implemented between the two shell portions, and this facilitates a more reliable sensing operation.

In particular, the pressure sensor pad 4 may be provided disposed between the body part and the one of the two shell portions 22a, 22b which does not move relative to the arm, to allow for stable measurements.

To implement this configuration, in some embodiments, one of the at least two shell portions may be provided having a greater stiffness (lower flexibility) than the other of the two shell portions. For example, the first shell portion 22a is provided having a greater stiffness than the second shell portion 22b.

Additionally or alternatively, in some embodiments, at both the first 32 and second 34 overlap regions, one of the two shell portions may be positioned radially atop the other. For example, the second shell portion 22b may be disposed radially above/atop the first 22a shell portion at both of the overlap regions. This therefore forms a configuration with a (radially) lower shell 22a and a (radially) upper shell 22b, or, in other words, a (radially) inner shell 22a and a radially outer shell 22b.

The example of Fig. 3-6 shows this configuration.

The first shell portion 22a in the example of Figs. 3-6 effectively forms a lower/inner shell portion, and the second shell portion 22b effectively forms an upper/outer shell portion 22b. The first shell portion 22a in this example has a higher stiffness than the second shell portion 22b. The first shell portion 22a carries the pressure sensor pad 4 in this example.

Positioning the sensor pad 4 on the lower, stiffer shell may be advantageous for several reasons. One advantage of placing the sensor pad in the middle of the lower shell is improved useability. This position makes it easy to attach the cuff in such a way that the sensor pad is aligned with the brachial artery area. In particular, for optimal measurement, it is best that, during operation, the sensor pad is no more than 4 cm offset from the position of the brachial artery, and more preferably no more than 2 cm offset.

Furthermore, there may be advantage in mounting the sensor pad to the shell portion which is stiffer so as to provide a maximally stiff support for thereby ensuring that movement of the body part tissue will be maximally absorbed by the sensor pad and loss of motion signal through absorption in the shell portion is minimized.

Preferably, the product of modulus of elasticity (Emod) and shell thickness is different for the two shell portions. Preferably, the first shell portion 22a carrying the pressure sensor pad 6 is stiffer than the second shell portion 22b. Preferably the difference in the product of Emod and shell thickness is in the range of 5-1000% and more preferably in the range of 30-500%.

The lower shell portion 22a which contains the sensor pad 6 preferably has an Emod in the range of 100 to 7000 MPa and more preferably 300-5000 MPa. The thickness of the lower shell portion 22a may be within a range of 0.5-3mm and more preferably in the range of 0.8-2 mm. The length of the upper shell portion 22b in the tangential direction may be in the range of 100 to 400 mm and more preferably in the range of 150 to 300 mm. The axial length of the lower shell portion 22a may preferably be between 100 mm and 200 mm and more preferably between 130 mm and 170 mm.

The upper shell portion 22b preferably has an Emod in the range of 100 to 5000 MPa and more preferably 500 to 1500 MPa. The thickness of the upper shell portion is preferably in the range 0.5-3 mm, and more preferably in the range of 0.8-2 mm.

As mentioned above, the apparatus includes a pressure actuator for applying a controllable pressure to the body part, for example for compressing the arm. The pressure actuator may include an inflatable bladder, wherein an inflation level of the bladder determines the applied pressure. However, it is also possible to employ other operating principles for the actuator. For instance, any other options to apply pressure on the arm can be used by e.g., electrical, or hydraulic or pneumatic means. Examples are a motorized hose clamp, or pneumatic cylinders.

Fig. 5 and Fig. 6 illustrate the cuff 20 with the actuator 52 in place. The actuator 52 is arranged to extend around the outside of the shell structure 22. The placement and structure of the actuator is shown only schematically; the radial thickness of the actuator may be thicker than that shown for example.

The actuator 52 in this example is arranged for wrapping around the body part when the cuff is worn, and in particular can be wrapped tangentially around the shell structure 22 when the cuff 20 is worn and can be unwrapped when the cuff is to be removed. The actuator may thus take the form of a body-part wrap structure which is foldable or rollable around the body part, similar to a typical NIBP cuff.

In other words, it may be said that the actuator 52 has a closed state and an open state, wherein, in the closed state, the actuator forms a closed loop around the central lumen, and wherein, in the open state, an opening is defined in a circumference of the actuator to permit insertion of the arm sideways into the cuff. Transition from the closed to open state comprises unrolling the actuator 52 to tangentially separate two ends of the actuator.

To ease the process of fitting the cuff to the patient, the pressure actuator 52 may be attached, fixedly or removably, to the shell structure. In some examples, the actuator may be attached to the shell structure 22 at only a specific limited set of positions. At the area of the sliding overlap 34 it is preferable that the actuator not be attached to one of the two shell portions, preferably the lower shell portion. This prevents the actuator from creating wrinkles as the ends of the shell portions slide over one another.

Fig. 6 shows the placement of the pressure actuator 52 relative to the shell structure 22 in more detail and also shows the regions across which the actuator is attached, fixedly or removably, to the shell structure 22. In particular, across region 62 the actuator 52 is fixated to the second shell portion 22b. Across region 64, the actuator 52 is fixated to the first shell portion 22a.

A first end 72 of the actuator is shown, and a second end 74 of the actuator is shown. It can be seen that the two ends of the actuator 52 overlap and are releasable from one another to allow the actuator to be unfolded.

Thus, it will be recognized that the pressure actuator 52 is for wrapping around the body part when the cuff is worn, around an outside of the shell structure 22, and wherein the actuator is attached at a first attachment zone 64 to an outside of the first shell portion 22a and is attached at a second attachment zone 62 to an outside of the second shell portion 22b. In the example of Fig. 6, the actuator is attached to the second shell portion 22b across its entire tangential extent. In other words, the second attachment zone 62 spans the whole of the second shell portion 22b. However, this is not essential. The second attachment zone may span only a sub-region of the second shell portion 22b. For example, in some embodiments, the second attachment zone spans a sub-region of the shell portion which fully encompasses the second overlap region 34. This helps avoid wrinkling at the second (sliding) overlap region.

The actuator 52 may comprise, in its unrolled state, a (length) section which incorporates an inflatable bladder and at least one (length) section having the form of a sheet without any bladder incorporated therein, wherein the actuator is for wrapping around the shell portion of the cuff with its length extending around the circumferential dimension of the cuff. The bladder portion of the actuator provides the source of pressure. The non-bladder portion aids in attachment of the actuator to the shell in a way that permits most effective coupling of pressure to the shell and arm, and reduces slippage.

For example, as illustrated in Fig .6, in some embodiments, a section of an inflatable bladder portion 55 of the actuator 52 is directly attached to the second shell portion 22b across the second attachment zone 62, a first non-bladder portion 54a of the actuator is attached to the first shell portion 22a across an end section of the shell located at the first overlap region 32, and a second non-bladder portion 54b is arranged to overlap a section of the inflatable bladder portion 55 when the actuator is wrapped around the shell structure 22 in use. In Fig. 6, arrow 76 shows a transition or junction point between the first non-bladder portion 54a of the actuator and a bladder portion 55 of the actuator, and arrow 78 shows a transition or junction point between the second non-bladder portion 54b of the actuator and the bladder portion 55 of the actuator. The second non-bladder portion 54b effectively performs an additional securement function. An attachment means, such as Velcro, may be provided for coupling the second non-bladder portion 54b of the actuator to an upper surface of the bladder-portion 55 of the actuator beneath. By pulling non-bladder portion 54b tight before attachment, it can be used to help retain the actuator secured around the shell structure when the cuff is in the closed state.

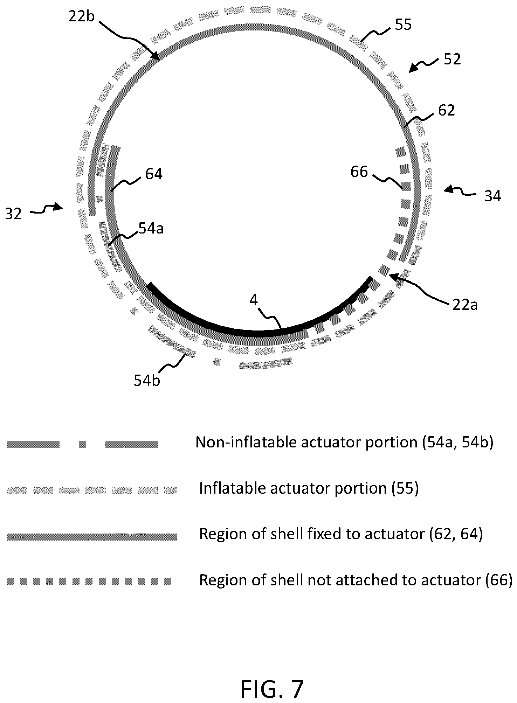

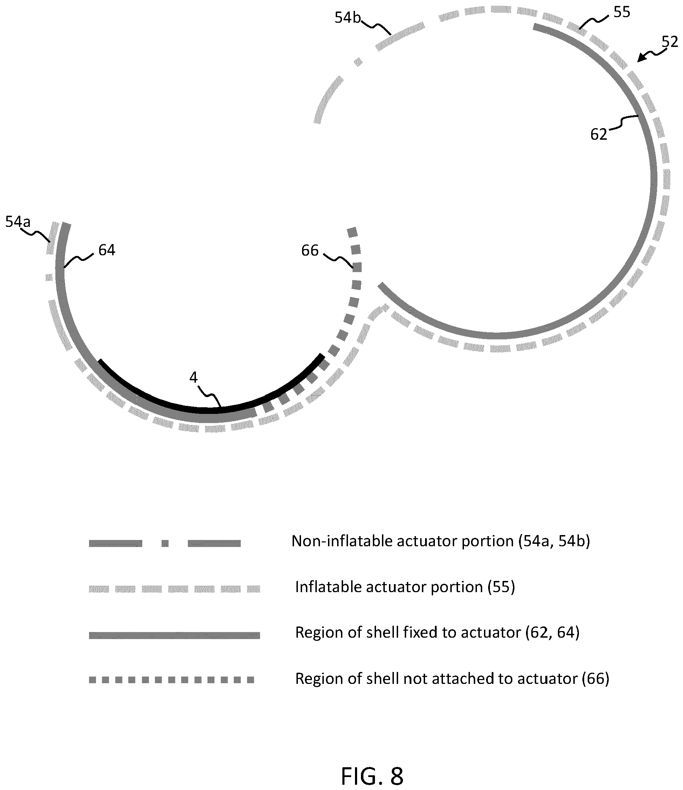

A further schematic illustration of the placement of the different portions of the pressure actuator 52 relative to the shell portions is provided in Fig. 7 and Fig. 8.

Fig. 7 shows the cuff in a closed state, with the pressure actuator 52 wrapped around the first 22a and second 22b shell portions. The different functional portions of the actuator are indicated with different dashed lines. The portion 55 of the pressure actuator incorporating an inflatable bladder is indicated, as well as the first 54a and 54b portions of the pressure actuator which do not incorporate an inflatable bladder. The first 22a and second 22b shell portions are indicated. For each shell portion, the section of the shell portion to which the pressure actuator 52 is fixedly attached is indicated with a solid line, while the section of the shell portion to which the pressure actuator is not attached is indicated with a dotted line. As indicated, in this example, the whole circumferential extent of the second shell portion 22b is fixedly attached to the pressure actuator 52, this attachment region forming the previously mentioned second attachment zone 62. Only one section of the first shell portion 22a is fixedly attached to the pressure actuator 52, this part corresponding to the previously mentioned first attachment zone 64. A further section 66 of the first shell portion 22a which is not attached to the actuator 52 is indicated. This section extends from an end of the first shell portion 22a located at the second overlap area 34 part way along the circumferential extension of the first shell portion.

Fig. 8 shows the same cuff as Fig. 7 in an open state.

It will be recognized that, in the closed state, the second non-bladder section 54b of the actuator 52 radially overlaps one sub-section of the bladder portion 55 of the actuator 52. It may attach or couple to the bladder portion 55 beneath using an attachment means such as Velcro.

In the illustrated example, the actuator has two functions: applying a pressure to the body part to compress the artery 8 and a secondary role of holding the sections 22a, 22b of the shell structure together radially when the cuff is worn. The first function of compression is the primary function.

To apply the pressure actuator 52 to the shell portions 22a, 22b it is preferable that the actuator layer 52 (e.g. comprising the inflatable bladder) is as thin as possible at the overlap area 32 where the shell portions are fixated to each other, i.e. the first overlap area 32. To this end, the tangential end of the actuator 52 may be attached to the first shell portion 22a at a location tangentially offset from the first overlap area. This may be done by gluing, but also other processes are possible i.e., ultrasonic welding or high frequency welding. In operation, the upper shell portion 22b will overlap the first area of the actuator 52 when the cuff is applied on a relatively small arm.

With regards to the actuator 52, it is preferable for measurement accuracy that the pressure-applying part extends fully around the body part (fully annularly) when the cuff is worn. For example, where the actuator comprises an inflatable bladder, the bladder containing portion 55 should extend fully around the body part so that pressure can be applied evenly.

Furthermore, it is preferable that the pressure-applying part of the actuator not wrinkle at the area of the sliding overlap 34. To enable this, and as mentioned previously, the actuator may be attached to the shell structure along a continuous attachment zone which spans the full tangential length of the second overlap region 34 (the sliding overlap). One possible attachment means for example is Velcro^{®}, which permits continuous attachment along an extended strip. Other possibilities include glue, and also ultrasonic welding or high frequency welding of the actuator to the shell structure.

Where the actuator 52 is of a form which comprises an inflatable bladder, the actuator may be arranged relative to the shell structure such that an air tube which supplies the bladder is not within the second (sliding) overlap area 34. Preferably it will be on the same side (behind) as the handle 72 of the inflatable bladder part of the cuff.

At least a portion of the actuator 52 may be permanently attached or fixated to the shell to ensure proper placement of the inflatable bladder of the actuator when the actuator is wrapped around the shells during use of the cuff and also to guarantee a particular relative placement of the shells to one another when the actuator is so wrapped. As mentioned above, the actuator plays a secondary role of holding the shell portions 22a, 22b together with a certain spatial relationship.

Each of the shell portions 22a, 22b may, by way of example, be made by a simple open-close injection-molding construction. As will be explained further below, in some embodiments, each of the shells may be formed from two different basic materials. This may improve friction characteristics, particularly at the sliding overlap region 34. It may also help to tune stiffness and wall thickness.

A relevant feature for implementing the sliding overlap at the second overlap region 34 is the static and the dynamic friction coefficient of the overlapping shell parts 22a, 22b. The dynamic friction coefficient is preferably in the range of 0.02 and 0.35 and more preferably in the range of 0.05 to 0.18, for providing a low friction. The static friction coefficient should preferably be close to the dynamic friction coefficient. For example, preferably the difference is less than 0.1 and more preferably less than 0.03 to reduce stick-slip effects of the sliding shell during a measurement.

With regards to material properties for the shell portions 22a, 22b, to achieve desirable stiffness and friction coefficients there are several options. One option is to use HDPE for the first shell portion 22a as well as for the second shell portion 22b. Other options for materials include PP, POM, and Polyimide. Optionally, additives such as silicon oil can be added to the material. It is also possible to use different materials for the first 22a and the second 22b shell portions. Options to realize this difference are for instance HDPE-PTFE, HDPE-Polyimide, POM-HDPE, HDPE-PP. Note that additives, such as silicone oil can be added to lower the overall friction coefficient.

To reduce the friction between the shell portions, it is possible to apply a foil on either the first and/or second shell portions. For instance, a PTFE foil or a Kapton (Polyimide) foil may be chosen, where the PTFE foil has the advantage of a low dynamic friction coefficient as well as a small difference between static and dynamic friction coefficients.

Another option is to use a structured interface at the sliding overlap of the second overlap region 34 to lower the friction. Preferably (when applicable) the hardest material (largest Elastic modulus) has the structured interface. Preferably the Ra value (roughness average of the surface, which is a measure of the average deviation of the surface from its mean line or centerline) is in the range of 0.03 to 20 µm and more preferably in the range of 0.1 to 5 µm.

Another option is to provide the first and/or second shell portions 22a, 22b as two-shot molded pieces, wherein the sliding overlap area 34 is a reduced thickness layer with preferably a thickness in the range of 0.05 mm to 0.6 mm and more preferably in the range of 0.2 mm to 0.3 mm. The length of the second shot material is preferably in the range of 30 mm to 120 mm and more preferably in the range of 60 mm to 80 mm.

In some embodiments, tangential edges the first shell portion 22a and/or the second shell portion 22b may be chamfered or rounded. This can be beneficial for a smooth sliding of the shells over one other. The chamfer may be an angle, α, at the free end of the shell, where said angle α is preferably in the range of 10 to 145° and more preferably in the range of 20 to 110°.

As mentioned above, preferably the lower shell portion 22a carries or comprises the pressure sensor pad 4. Preferably, the shell portion which contains the sensor pad 6 has an elastic modulus in the range of 100 to 7000 MPa and more preferably 300-5000 MPa. This thickness of this shell portion may be within the range 0.5-3mm and more preferably in the range of 0.8-2 mm. The length of the upper shell 22b in the tangential direction may be within the range 180 mm to 240 mm and more preferably 200 to 220 mm. The axial length of the lower shell 22a is preferably between 100 and 200 mm and more preferably between 130 and 170 mm. The upper shell part 22b preferably has an elastic modulus in the range of 100 to 5000 MPa and more preferably 500 to 1500 MPa. The thickness of the upper shell portion 22b may be in the range 0.5-3mm and more preferably in the range of 0.8-2 mm.

As mentioned above, in some embodiments, the apparatus includes the tissue pressure sensor for sensing a pressure between the cuff 20 and the body part when the cuff is worn, the tissue pressure sensor comprising a sensing pad 4 for disposal, in use, between the shell structure 22 and the body part. As mentioned above, in some embodiments, the tissue pressure sensor pad 4 is arranged so as to be disposed between the first shell portion 22a and the body part during use, and preferably wherein the first shell portion is stiffer than the second shell portion 22b and is radially beneath the second shell portion 22b.

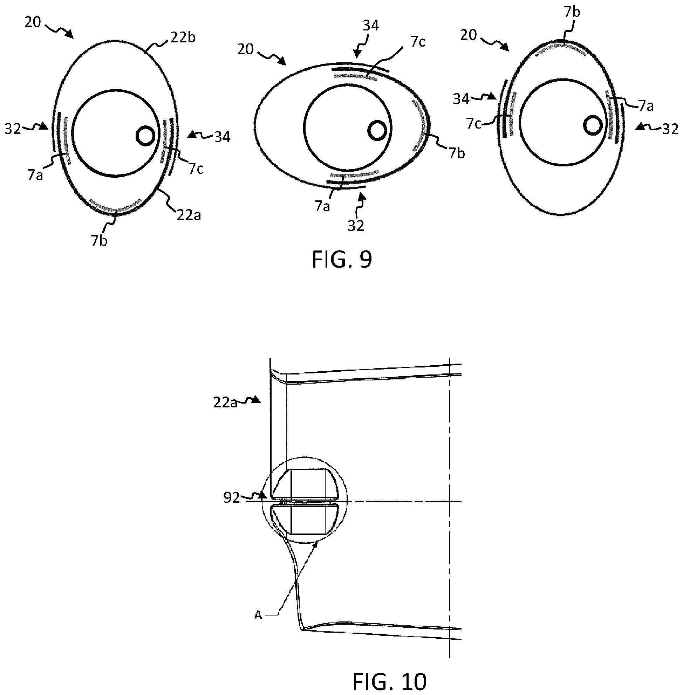

Fig. 9 illustrates three example positions 7a, 7b, 7c to place the sensor pad on the lower shell, any one of which may be acceptable. However, an optimal choice may be to position the sensor pad 4 in the middle of the lower shell, i.e. position 7b. This position in the lower shell makes it easier to apply to the cuff 20 around the arm so as to have the sensor pad aligned with the brachial artery. In particular, Fig. 7 (middle) shows the optimal placement of the cuff relative to the brachial artery and arm in the case that position 7b is chosen for the sensor pad.

As mentioned above, in some embodiments, the tissue pressure sensor is a hydraulic pressure sensor having a pressure sensor pad 4 which is fluidically coupled to a pressure transducer which transduces changes in fluid pressure inside the pad into a pressure measurement. The fluid connection between the pad 4 and the transducer may take the form of a fluid tube. To accommodate this, the shell portion which comprises or carries the pressure sensor pad 4, e.g. the first shell portion 22a, may include a tube guidance slot or channel within which the hydraulic tube can be fitted. This is illustrated in Fig. 10 which shows an interior side view of the first shell portion 22a, wherein the tube guidance slot 92 is shown.

This tube guidance 92 ensures that the tube will not be pressed into the skin during the measurement. This tube guidance takes the form of a slot or channel recessed radially into an interior surface of the first shell portion 22a such that the tube fits into this cavity and does not press onto the skin during operation. The length of the cavity may be between 1 and 30 mm and the width and depth may be in the range of 1 to 10 mm. The guidance may be fabricated for example by injection molding and can be integrated in one single mold. An additional benefit of this feature is that the hydraulic tube is held static during measurement, reducing the risk of artefacts caused by motion of the tube. This also means that it may be relatively easy to attach the sensor pad 4 onto the shell portion 4 during assembly, since it can be done at an exposed interior surface of the shell portion 22a.

In some embodiments, the distance of the sensor pad 4 measured from the distal end of the shell structure is preferably in the range of 10 mm to 45 mm and more preferably 20 mm to 30 mm.

In some embodiments, the shell structure, when in the closed state, forms a tubular shape having a non-uniform diameter, for example having a linearly tapering diameter, whereby the closed shell structure has a truncated conical shape to better fit the arm. Preferably, the conical angle is in the range of 1 to 15 degrees and more preferably in the range of 2 to 8 degrees.

Furthermore, although in the drawings, the shell structure is illustrated with a substantially circular cross-section, cross sectional shapes other than circular are also possible, such as elliptical.

The apparatus may further comprise a processing device comprising one or more processors.

The one or more processors may be adapted to receive a pressure signal from the tissue pressure sensor, and to apply one or more computation algorithms to the pressure signal to derive one or more hemodynamic parameter.

The one or more processors may additionally or alternatively be adapted to control a pressure applied by the pressure actuator in order to implement a measurement cycle. The pressure actuator may comprise an inflatable bladder, and wherein the control module is adapted to control an inflation/deflation cycle of the bladder. The apparatus may include a pressurized air source, such as an air pump, for use in inflating such a bladder.

The inventors have constructed test apparatuses in accordance with the above-described principles. According to one example, the first shell portion 22a was provided a 1.5 mm HDPE shell, while the second shell portion was provided as a 1 mm HDPE plate. On the first shell portion 22a was applied a thin Kapton foil to reduce both the dynamic friction coefficient and the static friction coefficient.

Using this example apparatus, hemodynamic parameters were measured. The same hemodynamic parameters were also measured using a known version of the shell backed cuff, in particular according to the example described in document EP2953528B1. For each cuff, the measurements were recorded for three different test persons (labelled VT3, VT5 and VT9). The results are shown in Table 1 below, where all important hemodynamic parameters are, for all three test persons, statistically equal. Note that the CO (cardiac Output) slightly deviates between the two cuffs, mainly due to a different (PR) pulse rate, which is physiological.

The cuff in accordance with an embodiment of the present invention is labelled A and the cuff in accordance with EP2953528B1 is labelled B.

**Table 1**

| | | SAP | | MAP | | DAP | | SV | | CO | | PR | |
|---|---|---|---|---|---|---|---|---|---|---|---|---|---|
| | | mean | std | mean | std | mean | std | mean | std | mean | std | mean | std |
| **Subject** | **Version** | | | | | | | | | | | | |
| VT3 | A | 127.02 | 4.18 | 91.17 | 3.44 | 69.61 | 3.26 | 86.39 | 3.33 | 6.50 | 0.56 | 75.13 | 3.90 |
| | B | 126.53 | 2.47 | 90.11 | 1.55 | 68.44 | 1.73 | 89.14 | 4.33 | 6.35 | 0.23 | 71.31 | 1.76 |
| VT4 | A | 127.97 | 1.43 | 94.48 | 3.55 | 73.48 | 4.36 | 83.66 | 8.67 | 6.54 | 0.68 | 78.15 | 1.19 |
| | B | 130.92 | 5.20 | 93.91 | 2.70 | 71.65 | 1.75 | 87.56 | 3.69 | 6.58 | 0.40 | 75.07 | 1.49 |
| VT5 | A | 154.53 | 7.07 | 113.62 | 10.94 | 87.97 | 11.41 | 73.54 | 5.16 | 4.70 | 0.47 | 63.78 | 1.49 |
| | B | 149.63 | 3.58 | 114.42 | 1.76 | 90.82 | 1.86 | 69.52 | 6.20 | 4.01 | 0.24 | 57.86 | 3.56 |

The results show that the hemodynamic parameters measured with the cuff A are statistically the same as those measured with the existing cuff version B. Accordingly, it is demonstrated that the advantages of the openable structure of the shell in embodiments of this invention do not lead to any loss in measurement quality.

In a further test, a second example cuff was constructed, wherein the first shell portion 22a comprised a 1 mm thick layer of POM material and the second shell portion 22b comprised a 1 mm thick layer of HDPE material. In testing, this exhibited similar results as the POM-HDPE shell system described above. Furthermore, this second example can be directly made from injection molding and does not need the application of a foil.

Although examples described above relate to an apparatus in which the shell structure comprises two shell portions, in further embodiments, the shell structure may comprise more than two shell portions, for instance three or more overlapping shell portions. Similar principles to those discussed above may be applied.

Some embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An apparatus for use in non-invasive hemodynamic parameter measurement, comprising:
a cuff (20) for extending around a body part (10) of a subject,
wherein the cuff comprises a pressure actuator (52) for applying a controllable pressure to the body part, and further comprises a shell structure (22) for extending around the body part and, when the cuff is worn, is disposed between the actuator and the body part;
wherein the shell structure (22) is configurable in each of a closed state and an open state,
wherein, in the closed state, the shell structure (22) forms a closed loop around a central lumen, the central lumen for receiving the body part (10) of a subject, and wherein, in the open state, an opening is defined in a circumference of the shell structure to permit insertion of the arm into the lumen;
wherein the shell structure is composed of at least a first and second shell portion (22a, 22b), each for defining a respective portion of a circumference of the shell structure when the shell structure is in the closed state, the shell portions (22a, 22b) overlapping tangentially in the closed state in at least a first overlap area (32) and a second overlap area (34);
wherein transition from the closed state to the open state comprises tangentially separating the shell portions (22a, 22b) at least at one of the overlap areas.

2. The apparatus of claim 1, further comprising a releasable securement means (42) at the first overlap area for, when secured, preventing lateral sliding between the first and second shell portions in a tangential direction at the first overlap area (32) when the shell is in the closed state.

3. The apparatus of claim 2, wherein the releasable securement means (42) permits securement of the first and second shell portions at the first overlap area (32) at a plurality of different relative tangential positions.

4. The apparatus of claim 3, wherein the securement means (42) comprises a hook-and-loop fastening.

5. The apparatus of any preceding claim, wherein, at least at the second overlap area (34), the first and second shell portions are freely laterally slidable in a tangential direction when the shell is in the closed state.

6. The apparatus of any preceding claim, wherein, when the cuff is in use on the body part, the pressure actuator (52) is arranged to radially support the shell structure against radial separation of the at least first and second shell portion.

7. The apparatus of any preceding claim, further comprising a sheet article (26) disposed, when the cuff is applied to the arm, between the shell structure (22) and a surface of the body part (10), and arranged for contacting the body part during use, wherein the shell structure (22) is slidable tangentially relative to the sheet article.

8. The apparatus of claim 7, wherein the sheet article (26) is attached to the shell structure (22), wherein the sheet article is arranged to extend continuously around a radially interior face of the shell structure between a first attachment point (28a) on the first shell portion (22a) and a second attachment point (28b) on the second shell portion (22b), and preferably wherein the first and second attachment points are located at the first overlap area (32).

9. The apparatus of claim 8, wherein the sheet article (26) extends uninterrupted across the second overlap area (34) and is discontinuous at the first overlap area (32), wherein a first end is attached to the first shell portion (22a) at the first overlap area (32) and the second end is attached to the second shell portion (22b) at the first overlap area (32).

10. The apparatus of any preceding claim, wherein one of the at least two shell portions (22a, 22b) has a greater stiffness than the other of the two shell portions, and optionally wherein the first and second shell portions are formed of different materials.

11. The apparatus of any preceding claim, wherein the shell structure includes a first shell portion (22a) and a second shell portion (22b), and wherein, at both the first (32) and second (34) overlap regions, the second shell portion (22b) is disposed radially atop the first shell portion (22b).

12. The apparatus of claim 11, wherein the second shell portion (22b) is less stiff than the first shell portion (22a).

13. The apparatus of any preceding claim, further comprising a tissue pressure sensor for sensing a pressure between the cuff (20) and the body part (10) when the cuff is worn, the tissue pressure sensor comprising a sensing pad (4) for disposal, in use, between the shell structure (22) and the body part, and
optionally wherein the second shell portion (22b) is less stiff than the first shell portion (22a), and wherein the tissue pressure sensor pad (6) is arranged so as to be disposed between the first shell portion (22a) and the body part during use.

14. The apparatus of any preceding claim, wherein the pressure actuator (52) is attached, fixedly or removably, to the shell structure (22).

15. The apparatus of claim 14, wherein the actuator has a closed state and an open state, wherein, in the closed state, the actuator forms a closed loop around the central lumen, and wherein, in the open state, an opening is defined in a circumference of the actuator to permit insertion of the arm into the lumen, wherein transition from the closed to open state comprises unrolling the actuator to tangentially separate two ends of the actuator.
